# EUROPÄISCHE PATENTSCHRIFT

(11) **EP 4 540 605 B1**
(45) Veröffentlichungstag und Bekanntmachung des Hinweises auf die Patenterteilung: **07.10.2026**
(21) Anmeldenummer: 23733267.1
(22) Anmeldetag: 14.06.2023
(51) Int. Cl.: G01N 33/00, A62B 7/00

(54) **VERFAHREN ZUM KALIBRIEREN VON SENSOREINHEITEN ZUM BESTIMMEN EINER KONZENTRATION EINES GASES IN EINEM GASGEMISCH**
METHOD FOR CALIBRATING SENSOR UNITS FOR DETERMINING A CONCENTRATION OF A GAS IN A GAS MIXTURE
PROCÉDÉ D'ÉTALONNAGE D'UNITÉS CAPTEURS POUR DÉTERMINER UNE CONCENTRATION D'UN GAZ DANS UN MÉLANGE GAZEUX

(30) Priorität: 15.06.2022 DE 102022115062
(43) Veröffentlichungstag der Anmeldung: 23.04.2025
(73) Patentinhaber: Bauer Kompressoren GmbH, 81477 München (DE)
(72) Erfinder: KAMPFL, Robert, 81379 München (DE); BURMEISTER, Thomas, 83679 Sachsenkam (DE); HUBER, Johannes, 86316 Friedberg (DE)
(74) Vertreter: Weickmann & Weickmann PartmbB
(86) Internationale Anmeldenummer: PCT/EP2023/065908
(87) Internationale Veröffentlichungsnummer: WO 2023/242239

(56) Entgegenhaltungen:
- DE-A1- 19 519 076
- US-A- 4 839 597
- US-A1- 2018 003 684

## Beschreibung

Die vorliegende Erfindung betrifft ein Verfahren zum Kalibrieren wenigstens einer Sensoreinheit, welche für ein Bestimmen einer Konzentration eines vorbestimmten Gases in einem Gasgemisch vorgesehen ist, sowie eine Analyseeinheit für Gasgemische, deren Steuereinheit dazu eingerichtet ist, ein derartiges Verfahren durchzuführen.

Es ist bekannt, dass bestimmte Gasgemische beispielsweise bei einer Abfüllung in Druckgasspeicher, wie etwa Atemluft in Atemluftzylinder, oder bei einer Entnahme aus solchen vor einer weiteren Verwendung ständig auf ihre Qualität sowie Zusammensetzung und insbesondere das Vorliegen von Verunreinigungen getestet werden müssen. Derartige Verunreinigungen dürfen hinsichtlich ihrer jeweiligen Konzentrationen in dem entsprechenden Gasgemisch vorgegebene Grenzwerte nicht überschreiten und es muss sichergestellt werden, dass in einem derartigen Fall eines Überschreitens eines Grenzwerts unmittelbar geeignete Gegenmaßnahmen getroffen werden, beispielsweise ein Betriebsstopp.

Hierzu werden Sensoreinheiten eingesetzt, die im oben genannten Beispiel zwischen einem Kompressor zum Verdichten des abzufüllenden Gasgemischs und dem Druckgasspeicher zwischengeschaltet sind und welche jeweils für ein Bestimmen einer Konzentration eines vorbestimmten Gases in dem Gasgemisch vorgesehen und eingerichtet sind. Hierbei können im eben beschriebenen Beispiel mehrere Sensoreinheiten vorgesehen sein, welche jeweils einen Bestandteil des Gasgemischs hinsichtlich seiner Konzentration überwachen können. Dies kann sich sowohl auf erwünschte als auch unerwünschte Bestandteile des Gasgemisches beziehen, sodass die Qualität und Zusammensetzung des Gasgemisches als Ganzes sowohl hinsichtlich seiner gewünschten Bestandteile als auch unerwünschter Verunreinigungen dauerhaft nachvollziehbar ist.

Es ist bekannt, dass bei der Verwendung derartiger Sensoreinheiten nach einem bestimmten Zeitintervall eine Nachjustierung der Sensorausgaben bzw. der von den Sensoreinheiten gelieferten Rohwerte erforderlich sein kann, um übliche Veränderungen an der Messstrecke und den Sensoren auszugleichen und die Genauigkeit der mittels der Sensoreinheiten bestimmten Konzentrationen dauerhaft zu gewährleisten. Wenngleich es sich hierbei, wie eben erwähnt, streng genommen um eine Justierung der entsprechenden Sensoreinheiten handelt, so hat sich in der Fachsprache für derartige Verfahren ebenfalls der Begriff der "Kalibrierung" etabliert, welcher im Folgenden durchgehend zur Beschreibung des erfindungsgemäßen Verfahrens verwendet werden wird.

Hierzu ist im Stand der Technik ein Abgleich von den durch die Sensoreinheiten gelieferten Rohwerten an zwei oder mehr definierten Punkten mit einem Gasgemisch durchgeführt worden, in welchem das entsprechende von der Sensoreinheit zu erfassende Gas als Bestandteil in einer bekannten Konzentration vorliegt, was üblicherweise als "Zweipunkt-Kalibration" bezeichnet wird. Ein Beispiel für eine derartige Zweipunkt-Kalibration ist aus der US 4 839 597 A bekannt, in welcher Sauerstoff-Konzentrationen von 21% und 100% verwendet werden. In diesem Zusammenhang sei ferner auf die DE 195 19 076 A1 hingewiesen, welche eine Einpunkt-Kalibrierung bei 21 % Sauerstoff vorschlägt. Der Vollständigkeit halber sei ferner auf die US 2018/003684 A1 hingewiesen, welche eine Kalibration bei mehr als zwei Konzentrationen offenbart.

Hierbei sind jedoch die laufenden Kosten zur Kalibrierung bzw. Justierung derartiger Sensoreinheiten sehr hoch, da entsprechende Kalibrier-Gasgemische mit einer definierten Konzentration des zu erfassenden Gases sehr teuer sind und das Verfahren von geschultem Servicepersonal durchgeführt oder wenigstens überwacht werden muss. Es besteht demzufolge ein Bedarf nach einem Verfahren und einer Analyseeinheit, mit welchen eine derartige Kalibrierung einfacher und unter Einsparung von Kosten durchgeführt werden kann, wobei jedoch weiterhin sichergestellt werden soll, dass nach der Kalibrierung die entsprechende Sensoreinheit immer noch ausreichend genaue Daten liefert.

Zur Lösung dieser Aufgabe wird erfindungsgemäß ein Verfahren zum Kalibrieren wenigstens einer Sensoreinheit vorgeschlagen, welche für ein Bestimmen einer Konzentration eines vorbestimmten Gases in einem Gasgemisch vorgesehen ist, wobei eine Grenzwert-Konzentration vorgegeben ist, welche durch das vorbestimmte Gas in dem Gasgemisch nicht überschritten werden darf, umfassend ein anfängliches Kalibrieren der Sensoreinheit bei wenigstens zwei vorbestimmten Konzentrationen des vorbestimmten Gases in einem jeweiligen Kalibrier-Gasgemisch, wenigstens umfassend die Schritte eines Ermittelns einer anfänglichen Nullpunkt-Ausgabe der Sensoreinheit mit einem ersten Kalibrier-Gasgemisch, in welchem die Konzentration des vorbestimmten Gases im Wesentlichen Null beträgt, eines Ermittelns einer weiteren Ausgabe der Sensoreinheit mit einem zweitem Kalibrier-Gasgemisch, in welchem die Konzentration des vorbestimmten Gases größer oder kleiner als die Grenzwert-Konzentration ist, und eines Bildens einer Ausgabe-Gerade der Sensoreinheit anhand der anfänglichen Nullpunkt-Ausgabe und der weiteren Ausgabe, wobei die Ausgabe-Gerade eine anfängliche Geradensteigung aufweist. Ferner umfasst das erfindungsgemäße Verfahren zu einem späteren Zeitpunkt ein erneutes Kalibrieren, umfassend die Schritte eines Ermittelns einer Grenzwert-Ausgabe der Sensoreinheit mit einem Grenzwert-Gasgemisch, in welchem die Konzentration des vorbestimmten Gases im Wesentlichen der Grenzwert-Konzentration entspricht, und eines Bildens einer aktualisierten Ausgabe-Gerade der Sensoreinheit anhand der Grenzwert-Ausgabe und der anfänglichen Nullpunkt-Ausgabe oder anhand der Grenzwert-Ausgabe und der anfänglichen Geradensteigung.

Dementsprechend wird erfindungsgemäß lediglich bei dem anfänglichen Kalibrieren der wenigstens einen Sensoreinheit eine Zweipunkt-Kalibrierung durchgeführt, in welcher einerseits eine Nullpunkt-Ausgabe der Sensoreinheit und andererseits eine weitere Ausgabe der Sensoreinheit bei einer höheren Konzentration des zu ermittelnden Gases in dem Gasgemisch bestimmt wird, wobei anhand dieser beiden Messpunkte dann die ursprüngliche Ausgabe-Gerade der Sensoreinheit gebildet wird. Hierbei kann vorzugsweise das zweite Kalibrier-Gasgemisch derart gewählt werden, dass die Konzentration des vorbestimmten Gases über dem Grenzwert liegt, um jeweils einen Datenpunkt unterhalb und oberhalb des Grenzwerts zur Verfügung zu haben und eine geeignete Ausgabe-Gerade aufstellen zu können. An dieser Stelle sei bereits darauf hingewiesen, dass das Ausgabeverhalten derartiger Sensoreinheiten nicht unbedingt streng linear sein muss, aber in aller Regel durch eine Gerade ausreichend gut angenähert werden kann. Falls notwendig, können im regulären Betrieb der Sensoreinheit dann zusätzliche Sicherheitsfaktoren eingeführt werden, um die gemessene Konzentrationswerte korrigiert werden, so dass ein Überschreiten der geforderten Grenzwerte auch an dieser Stelle noch einmal mit noch höherer Sicherheit ausgeschlossen werden kann.

Weiterhin sei darauf hingewiesen, dass das Ermitteln der Nullpunkt-Ausgabe auch mit einem ersten Kalibrier-Gasgemisch stattfinden kann, welches das vorbestimmte Gas in einer geringen Konzentration enthält, beispielsweise wenn ein Bereitstellen eines entsprechenden Gasgemischs ganz ohne das entsprechende Gas wirtschaftlich oder technisch zu aufwendig oder gar unmöglich wäre. Dementsprechend ist der Ausdruck "im Wesentlichen Null" im Zusammenhang mit einer Konzentration eines Gases in einem Gasgemisch im Rahmen der vorliegenden Erfindung derart zu verstehen, dass hier ein möglichst kleiner Wert anzustreben sein soll, welcher technisch und wirtschaftlich sinnvoll zu erreichen ist und einen ausreichenden Abstand zu der Konzentration des entsprechenden Gases in dem zweiten Kalibrier-Gasgemisch ermöglicht.

Bei dem anschließenden erneuten Kalibrieren der wenigstens einen Sensoreinheit wird dem hingegen erfindungsgemäß auf eine sog. "Grenzwert-Einpunkt-Kalibrierung" zurückgegriffen, bei welcher im Wesentlichen an demjenigen Punkt des Konzentrationsspektrums des entsprechenden Gases die Ausgabe der Sensoreinheit ermittelt wird, an welchem sich der Grenzwert dieses Gases in dem während eines regulären Betriebs der Sensoreinheit zu überprüfenden Gasgemisch befindet.

Auf Grundlage dieser einzelnen Ermittlung der Grenzwert-Ausgabe bei einem erneuten Kalibrieren der Sensoreinheit kann demzufolge sichergestellt werden, dass gerade an diesem Grenzwert der Konzentration des entsprechenden Gases in dem Gasgemisch die Sensorausgabe mit einer möglichst hohen Genauigkeit bekannt ist, während dementsprechend beispielsweise an einem oberen Ende des möglichen Messbereichs der entsprechenden Sensoreinheit größere Ungenauigkeiten in Kauf genommen werden können, da davon ausgegangen wird, dass an dieser Stelle eine große Entfernung zu dem wesentlichen Grenzwert vorliegt und demzufolge an dieser Stelle des Konzentrationsspektrums keine hohe Genauigkeit benötigt wird. Letzten Endes beruht die vorliegende Erfindung auf der Erkenntnis, dass durch eine Sensoreinheit in einem derartigen Szenario im Wesentlichen lediglich festzustellen ist, ob ein vorbestimmter Grenzwert eines gasförmigen Bestandteils eines Gasgemisches überschritten oder unterschritten ist und demzufolge das Gasgemisch für seinen vorgesehenen Verwendungszweck geeignet ist oder nicht.

Demzufolge besteht erfindungsgemäß sowohl die Möglichkeit, anhand der bei dem erneuten Kalibrieren ermittelten Grenzwert-Ausgabe und der anfänglichen Nullpunkt-Ausgabe der Sensoreinheit die aktualisierte Ausgabe-Gerade zu bilden, was bei einer Veränderung der Sensor-Ausgabe im Laufe der Zeit zu einer geänderten Geradensteigung der Ausgabe-Gerade führen wird, während andererseits ebenfalls denkbar ist, die ursprünglich ermittelte anfängliche Geradensteigung und die während des erneuten Kalibrierens erfasste Grenzwert-Ausgabe zum Bilden der aktualisierten Ausgabe-Gerade zu verwenden, was demzufolge unter anderem die Nullpunkt-Ausgabe an der Ausgabe-Gerade verschieben wird.

Hierbei handelt es sich bei den Ausgaben derartiger Sensoreinheiten typischerweise um elektrische Spannungen oder Ströme, von welchen erwartet wird, dass sie in ihren vorgesehenen Messbereichen eine wenigstens näherungsweise Linearität ihrer Ausgaben bezüglich der Konzentration des zu ermittelnden Gases aufweisen. Ferner sei darauf hingewiesen, dass das erneute Kalibrieren im Rahmen der vorliegenden Erfindung nach einer vorbestimmten Zeit bzw. im Rahmen einer vorbestimmten Zeitperiode oder auch wiederholt bei einer jeweiligen erneuten Inbetriebnahme der entsprechenden Sensoreinheit stattfinden kann.

In jedem Fall können durch das erfindungsgemäße Verfahren erhebliche Einsparungen erzielt werden, da für das erneute Kalibrieren lediglich ein einzelnes Grenzwert-Gasgemisch benötigt wird, verglichen mit wenigstens zwei Kalibrier-Gasgemischen bei aus dem Stand der Technik bekannten Zweipunkt-Kalibrierungsverfahren.

Da sich über die Lebensdauer der hier betrachteten Sensoreinheiten erhebliche Änderungen ihrer Sensitivität bzw. ihrer Sensorausgaben abhängig von zu bestimmenden Konzentrationen ergeben können, kann das erfindungsgemäße Verfahren nach dem Bilden der aktualisierten Ausgabe-Gerade ferner einen Schritt eines Überprüfens umfassen, ob die Geradensteigung der aktualisierten Ausgabe-Gerade und/oder ein Nullpunkt-Wert der aktualisierten Ausgabe-Gerade in einem vorbestimmten Wertebereich liegen. Auf diese Weise können beispielsweise Fälle abgefangen werden, in welchen die Sensor-Ausgabe in einem derartigen Maß zurückgegangen ist, dass bei einem Bilden einer aktualisierten Ausgabe-Gerade die anfängliche Nullpunkt-Ausgabe größer ist als die neu ermittelte Grenzwert-Ausgabe, was zu einer negativen Geradensteigung führen würde, die wiederum zu fehlerhaften Ergebnissen bei der Überwachung des Grenzwerts des entsprechenden Gases im Gasgemisch im regulären Betrieb der Sensoreinheit führen würde. Durch eine solche Plausibilisierung der aktualisierten Ausgabe-Gerade hinsichtlich ihrer Steigung und/oder ihres Nullpunkt-Werts können demzufolge derartige Probleme von vornherein ausgeschlossen werden und falls einer der genannten Werte nicht in dem vorbestimmten Wertebereich liegt, können entsprechende Gegenmaßnahmen getroffen werden, beispielsweise ein Ausgeben einer Warnung oder ein Stilllegen der entsprechenden wenigstens einen Sensoreinheit.

Ferner kann erfindungsgemäß in dem zweiten Kalibrier-Gasgemisch das vorbestimmte Gas mit einer Konzentration vorliegen, welche einer oberen Grenze des vorgesehenen Messbereichs der entsprechenden Sensoreinheit entspricht. Auf diese Weise werden bei dem anfänglichen Kalibrieren der Sensoreinheit die Nullpunkt-Ausgabe und eine möglichst weit von diesem Nullpunkt entfernte weitere Ausgabe der Sensoreinheit ausgewertet, was dementsprechend zu einer möglichst präzisen anfänglichen Ausgabe-Geraden führt.

Ferner kann das anfängliche Kalibrieren ein Ermitteln mehrerer weiterer Ausgaben mit unterschiedlichen Gasgemischen umfassen, in welchen die Konzentration des vorbestimmten Gases jeweils unterschiedlich und größer Null ist, sowie ein Bilden einer Ausgleichs-Ausgabe-Gerade der Sensoreinheit anhand der anfänglichen Nullpunkt-Ausgabe und der mehreren weiteren Ausgaben. Auch durch diese Maßnahme kann eine möglichst präzise anfängliche Ausgabe-Gerade der Sensoreinheit ermittelt werden, wobei durch das Aufnehmen mehrerer weiterer Ausgaben mit unterschiedlichen Gasgemischen statistische und systematische Schwankungen verringert werden. Zur Bildung der entsprechenden Ausgleichs-Ausgabe-Gerade können anschließend unterschiedliche Verfahren zum Einsatz kommen, wie beispielsweise eine Linearisierung mittels kleinster Quadrate oder eine angepasste Variante hiervon, in welcher beispielsweise der Nullpunkt fest gewählt oder stärker gewichtet werden kann.

Weiterhin kann in dem erfindungsgemäßen Verfahren während des anfänglichen und/oder erneuten Kalibrierens gleichzeitig eine Mehrzahl von Sensoreinheiten kalibriert werden, indem die jeweiligen Gasgemische eine entsprechende Mehrzahl von zu bestimmenden Gasen in geeigneten Konzentrationen enthalten. Dies bedeutet in der Praxis, dass eine geringere Anzahl von für die Kalibrierung einzusetzenden Gasgemischen bereitgestellt werden müssen, beispielsweise indem ein erstes Kalibrier-Gasgemisch vorgesehen wird, in welchem eine Mehrzahl von durch einzelne Sensoreinheiten zu erfassenden vorbestimmten Gasen jeweils in einer Konzentration von Null vorliegen, sowie ein zweites Kalibrier-Gasgemisch, in welchem die entsprechenden Gase bei einer jeweiligen höheren Konzentration vorliegen, welche jeweils das Bilden einer anfänglichen Ausgabe-Gerade für jede Sensoreinheit und jedes der zu erfassenden Gase in dem Gasgemisch ermöglichen.

Hierdurch kann in Systemen, in welchen eine Mehrzahl entsprechender Sensoreinheiten integriert vorgesehen sind, eine erhebliche Einsparung von Kosten und Aufwand erzielt werden, es müssen lediglich die entsprechenden Sensorausgaben in einer geeigneten Weise zwischengespeichert und anschließend verarbeitet werden. Dabei ist lediglich darauf zu achten, dass die zu kalibrierenden Sensoreinheiten keine Querempfindlichkeiten für mehrere der in dem Gasgemisch enthaltenen Bestandteile aufweisen, dies ist jedoch selbstverständlich abhängig von den Typen von vorbestimmten Gasen sowie der jeweiligen Bauart der einzelnen Sensoreinheiten. Da derartige Querempfindlichkeiten jedoch für in derartigen Anwendungen verwendete Sensoreinheiten bekannt sind, kann für entsprechende Kalibrierungsverfahren jeweils eine optimale Kombination von gasförmigen Bestandteilen in den einzelnen Kalibrier-Gasgemischen bzw. Grenzwert-Gasgemischen gesorgt werden, wobei vielerlei mögliche Kombinationen denkbar sind, zum Beispiel ein völlig gereinigtes Gas, in welchem wie angesprochen keiner der zu erfassenden Bestandteile überhaupt vorliegt, und/oder eine Mehrzahl von Grenzwert-Gemischen mit unterschiedlichen Bestandteilen, soweit diese in einer sinnvollen Weise herzustellen und mit den eingesetzten Typen von Sensoreinheiten zu verwenden sind.

In der Praxis kann das erfindungsgemäße Verfahren insbesondere dann Anwendung finden, wenn es sich bei dem Gasgemisch wie bereits weiter oben angedeutet um Atemluft handelt und/oder das vorbestimmte Gas CO, CO₂ oder O₂ ist. Für in Atemluftzylinder abzufüllende Atemluft gilt insbesondere die Norm DIN EN 12001, welche entsprechende Grenzwerte für die genannten gasförmigen Bestandteile vorgibt, beispielsweise 500 ppm für CO₂ und 5 ppm für CO. Diese genannten Werte entsprechen demzufolge den oben bereits mehrfach diskutierten Grenzwerten, bei welchen die erneute Kalibrierung der entsprechenden Sensoreinheiten im Rahmen der Grenzwert-Einpunkt-Kalibrierung in diesem Beispiel stattzufinden hätte.

Weiterhin kann das erfindungsgemäße Verfahren nach dem anfänglichen Kalibrieren und/oder dem erneuten Kalibrieren ein Prüfen der Kalibrierung mit einem Prüf-Gasgemisch umfassen, in welchem die Konzentration des vorbestimmten Gases geringfügig über der Grenzwert-Konzentration liegt. Auf diese Weise kann sichergestellt werden, dass auch bereits eine kleine Überschreitung des entsprechenden Grenzwerts zuverlässig festgestellt wird und demzufolge die entsprechende Sensoreinheit ihre Aufgabe in der vorgesehenen Weise erfüllt. In einem konkreten Beispiel kann die Konzentration des vorbestimmten Gases in dem Prüf-Gasgemisch um etwa 10-30% über der entsprechenden Grenzwert-Konzentration liegen, also im Fall von CO in Atemluft bei 6ppm verglichen mit dem Grenzwert von 5ppm.

Weiterhin kann das erfindungsgemäße Verfahren ein Überwachen einer Durchflussrate des jeweiligen Gasgemisches durch die wenigstens eine Sensoreinheit umfassen. Dieser weitere Erfindungsaspekt hat den Hintergrund, dass in der Regel bei der Kalibrierung derartiger Sensoreinheiten Gasdosen verwendet werden, da diese leicht zu transportieren sind und eine ausreichende Menge Gas für eine entsprechende Kalibrierung enthalten. Die in diesem Zusammenhang verwendeten handelsüblichen Druckminderer für solche Gasdosen haben jedoch den Nachteil, dass der Durchfluss durch den Dosendruck bestimmt wird und es demzufolge bei Kalibrierungen vorkommen kann, dass der Durchfluss nicht den späteren Betriebsbedingungen der zu kalibrierenden Sensoreinheit in einem Produktivbetrieb davon entspricht. Demzufolge könnte bei einer Abweichung von dem vorgesehenen Durchfluss die Kalibrierung ungenau werden und die entsprechende Sensoreinheit bei einer möglicherweise stattfindenden nachfolgenden Prüfung durchfallen.

Konkret könnte zum Durchführen dieses Verfahrensschritts über einen gemessenen Differenzdruck zwischen einem verwendeten Schlauchsystem und der Umgebungsluft der Durchfluss durch die entsprechende Sensoreinheit überwacht werden, sodass zu jedem Zeitpunkt der Kalibrierung bekannt ist, welcher Durchfluss im Sensor momentan vorhanden ist.

Hierbei könnte bei einem Unterschreiten und/oder Überschreiten einer jeweiligen Grenzwert-Durchflussrate für einen vorbestimmten Zeitraum eine Warnung ausgegeben werden oder ein automatischer Abbruch der Kalibrierung erfolgen. Das Vorsehen dieses vorbestimmten Zeitraums dient dem Zweck, dass nicht bei jeder kurzzeitigen Unter- oder Überschreitung des definierten Toleranzbereichs ein Abbruch erfolgen würde, was das Verfahren deutlich ineffizienter machen würde. Dementsprechend könnten beispielsweise Werte von ca. 10 Sekunden vorgesehen sein, nach denen die Kalibrierung abgebrochen würde, wenn die Grenzwert-Durchflussrate über- oder unterschritten würde.

Weiterhin betrifft die vorliegende Erfindung eine Analyseeinheit für Gasgemische, umfassend wenigstens eine Sensoreinheit, welche dazu eingerichtet ist, Sensordaten auszugeben, welche eine Konzentration eines vorbestimmten Gases in einem Gasgemisch repräsentieren, und eine Steuereinheit, welche mit der wenigstens einen Sensoreinheit betriebsmäßig gekoppelt und in einem Arbeitsmodus dazu eingerichtet ist, anhand der Sensordaten die Konzentration des vorbestimmten Gases zu ermitteln, wobei die Steuereinheit ferner dazu eingerichtet ist, zwischen dem genannten Arbeitsmodus und einem Kalibriermodus umschaltbar zu sein und in dem Kalibriermodus die Schritte des oben beschriebenen erfindungsgemäßen Verfahrens durchzuführen. Hierbei können unterschiedliche Automatisierungsgrade der Steuereinheit bzw. des Verfahrens zum Kalibrieren der wenigstens einen Sensoreinheit denkbar sein, beispielsweise kann vorgesehen sein, einen menschlichen Bediener die einzelnen Verfahrensschritte jeweils einzeln auslösen zu lassen oder auch das Verfahren weitestgehend automatisch durchzuführen, wobei ein menschlicher Bediener lediglich eine überwachende Funktion zu übernehmen hat.

Weiterhin kann die Steuereinheit der Analyseeinheit dazu eingerichtet sein, in dem Arbeitsmodus bei einem Feststellen eines Überschreitens einer Grenzwert-Konzentration des vorbestimmten Gases der wenigstens einen Sensoreinheit einen Steuerbefehl zum Öffnen eines Spülventils oder eines Abschaltens eines Kompressors auszugeben. Somit wird sichergestellt, dass beispielsweise im oben angeführten Beispiel eines Abfüllens von Atemluft bei einem Überschreiten eines der einzuhaltenden Grenzwerte sofort die Befüllung des entsprechenden Atemluftzylinders gestoppt wird und demzufolge ein Abfüllen von kontaminierter Atemluft zuverlässig vermieden wird.

Weiterhin kann die Analyseeinheit mit einem Gasspeicher bereitgestellt sein, welcher ein Prüf-Gasgemisch enthält, in welchem das wenigstens eine vorbestimmte Gas geringfügig oberhalb seiner Grenzwert-Konzentration vorliegt, und die Steuereinheit kann dazu eingerichtet sein, nach einer vorbestimmten Zeitspanne oder bei einer erneuten Inbetriebnahme der Analyseeinheit automatisch in einen Prüfmodus umzuschalten und eine Prüfung der wenigstens einen Sensoreinheit mithilfe des in dem Gasspeicher enthaltenen Prüf-Gasgemischs durchzuführen. Hierbei kann die Inbetriebnahme der Analyseeinheit beispielsweise im Zusammenhang stehen mit einer Inbetriebnahme eines Kompressors, dessen ausgegebenes Gas die Analyseeinheit überwacht.

Durch diese automatisierte Lösung, die nach einem vorgebbaren Intervall die Sensorik automatisch mit dem Prüf-Gasgemisch beaufschlagt und somit die wenigstens eine Sensoreinheit auf ihre Funktion prüft, können Wartungskosten eingespart und der Einsatz von Servicetechnikern vermieden werden. Hierbei erfolgt die entsprechende Ansteuerung direkt über die elektronische Steuereinheit des Systems, und nach Ablauf des in der entsprechenden Software eingestellten Prüfintervalls kann die Verbindung zu einer aktuellen Druckquelle, wie beispielsweise dem schon mehrfach angesprochenen Kompressor oder auch einem zu überprüfenden Atemluftzylinder, getrennt werden und ein entsprechendes Magnetventil zu dem Gasspeicher, also beispielsweise einer Prüfgasflasche, geöffnet werden. Hierdurch fließt das Prüf-Gasgemisch mit einer voreingestellten Durchflussrate durch die wenigstens eine zu prüfende Sensoreinheit und es kann durch die Steuereinheit überwacht werden, ob die ausgegebenen Sensorwerte in einem vorbestimmten Toleranzbereich liegen. Sollte dies nicht der Fall sein, so kann ein Abschalten des Kompressors oder die Ausgabe einer entsprechenden Warnung erfolgen. Um zu verhindern, dass während dieser automatischen Sensorprüfung ungeprüftes Gasgemisch abgefüllt wird, kann je nach Betriebsweise des Systems der Kompressor abgeschaltet werden oder ein zwischengeschaltetes Spülventil geöffnet werden.

Da während des anfänglichen oder erneuten Kalibrierens und/oder des Prüfens das beispielsweise von einem durch die wenigstens eine Sensoreinheit überwachten Kompressor für eine Abfüllung verdichtete Gasgemisch nicht durch die Sensoreinheit überwacht werden kann, können erfindungsgemäß Maßnahmen getroffen werden, um das Abfüllen von derart nicht überwachtem Gasgemisch zu verhindern. Insbesondere kann zu diesem Zweck während des anfänglichen oder erneuten Kalibrierens und/oder des Prüfens der entsprechende Kompressor abgeschaltet werden, ein Spülventil zwischen dem Kompressor und einem entsprechenden Druckgasspeicher geöffnet werden und/oder eine Anzeige für einen Bediener angepasst werden, so dass dieser über das momentane Durchführen des entsprechenden Verfahrensschritts informiert ist und ggf. manuell entsprechende Maßnahmen einleiten kann.

Dementsprechend entspricht der eben beschriebene Prüfmodus prinzipiell dem Arbeitsmodus der Analyseeinheit, hat jedoch bei einem Überschreiten eines vorgegebenen Grenzwerts nicht zur Folge, dass eine Stilllegung des Systems erfolgen muss, sondern dass vielmehr die Funktion des Systems verifiziert werden kann.

Wie bereits weiter oben angesprochen, kann die oder wenigstens eine der Sensoreinheiten eine elektrochemische Sensoreinheit sein, wobei derartige Sensoreinheiten derart funktionieren, dass sie eine chemische Substanz enthalten, welche mit dem zu analysierenden vorbestimmten Gas reagiert, und hierdurch ein elektrisches Signal erzeugen.

Weiterhin betrifft die vorliegende Erfindung einen Kompressor zum Verdichten von Atemluft für eine Abfüllung in einen Druckgasspeicher, umfassend eine Analyseeinheit der eben beschriebenen Art, welche dazu eingerichtet ist, während eines Betriebs des Kompressors die ausgegebene komprimierte Atemluft auf eine Konzentration von wenigstens einem vorbestimmten Gas zu überwachen. Hierbei kann die Steuereinheit der Analyseeinheit mit einer Steuereinheit des Kompressors betriebsmäßig gekoppelt oder integral mit ihr gebildet sein, um eine erhöhte Integration des Systems zu erzielen und ggf. beispielsweise bei einem Überschreiten eines Grenzwerts unmittelbar eine Abschaltung des Kompressors in einer effizienten Weise vornehmen zu können.

Weitere Merkmale und Vorteile der vorliegenden Erfindung werden aus der nachfolgenden Beschreibung von Ausführungsformen davon noch deutlicher, wenn diese zusammen mit den beiliegenden Figuren betrachtet wird. Diese zeigen im Einzelnen:
- Figur 1: ein schematisches Blockdiagramm einer erfindungsgemäßen Vorrichtung;
- Figur 2: ein Flussdiagramm eines erfindungsgemäßen Verfahrens; und
- Figur 3: mehrere Diagramme zur Verdeutlichung des Verfahrens aus Figur 2.

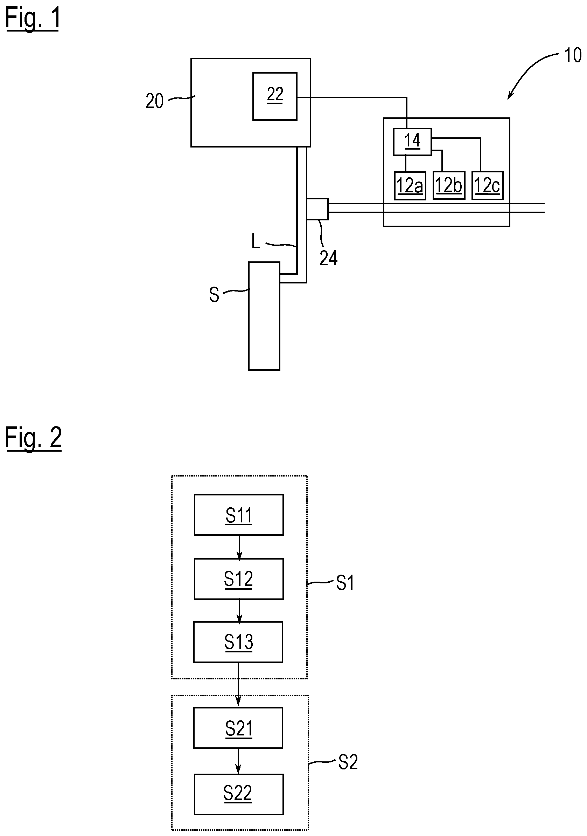

In Figur 1 ist zunächst einmal eine erfindungsgemäße Analyseeinheit für Gasgemische schematisch dargestellt und ganz allgemein mit dem Bezugszeichen 10 bezeichnet. Die Analyseeinheit 10 umfasst eine Mehrzahl von Sensoreinheiten 12a bis 12c, welche beispielsweise zu einer jeweiligen Messung einer Konzentration von CO, CO₂ und O₂ in für eine Abfüllung in Atemluftzylinder vorgesehener Atemluft vorgesehen und eingerichtet sind. Ferner umfasst die Analyseeinheit 10 eine Steuereinheit 14, welche mit den Sensoreinheiten 12a bis 12c jeweils betriebsmäßig gekoppelt ist und die entsprechenden Sensorausgaben in einem Arbeitsmodus verarbeiten kann, um eine Überwachung von vorgegebenen Grenzwerten der entsprechenden Bestandteile der abzufüllenden Atemluft vorzunehmen.

Weiterhin zeigt Figur 1 einen Kompressor 20, der die bereits angesprochene verdichtete und abzufüllende Atemluft liefert und ebenfalls eine Steuereinheit 22 umfasst, welche in der gezeigten Ausführungsform mit der Steuereinheit 14 der Analyseeinheit 10 betriebsmäßig gekoppelt ist. Die von dem Kompressor 20 verdichtete Atemluft wird im regulären Betrieb davon in einen als Druckgasspeicher wirkenden Atemluftzylinder S mittels eines lediglich schematisch dargestellten Leitungssystems L abgefüllt, wobei über einen Druckminderer 24 ein Teil der verdichteten Atemluft entnommen und der Analyseeinheit 10 zu einer Analyse mittels der Sensoreinheiten 12a bis 12c zugeführt wird. In einer alternativen Variante der gezeigten Vorrichtung könnte auf den Druckminderer 24 auch verzichtet werden, um die Sensoreinheiten 12a bis 12c direkt mit Hochdruck zu beaufschlagen. Es versteht sich ferner, dass insbesondere das Leitungssystem L weitere hier nicht gezeigte Komponenten umfassen kann, beispielsweise eine Gas-Trocknungseinheit oder Sicherheitsventile, die jedoch im Rahmen der vorliegenden Erfindung nicht weiter von Belang sind.

Bei einem Überschreiten eines vorbestimmten Grenzwerts einer Konzentration von einem der genannten Bestandteile der abzufüllenden Atemluft und einer entsprechenden Erfassung durch die Sensoreinheiten 12a bis 12c kann mittels der Steuereinheit 14 eine Warnung ausgegeben werden und/oder unmittelbar eine Anweisung zum Einstellen des Betriebs des Kompressors 20 an dessen Steuereinheit 22 übermittelt werden. Alternativ könnte auch ein nicht dargestelltes Spülventil geöffnet werden, sodass die komprimierte Atemluft ebenfalls nicht in den Atemluftzylinder S gefüllt wird, jedoch der Betrieb des Kompressors 20 nicht gestoppt werden muss. Ferner kann in bestimmten Ausführungsformen der vorliegenden Erfindung die Analyseeinheit 10 auch direkt in dem Kompressor 20 integriert sein, so dass die beiden Steuereinheiten 14 und 22 auch integral gebildet sein könnten.

Zum erfindungsgemäßen Kalibrieren der Sensoreinheiten 12a bis 12c kann nunmehr ein Verfahren gemäß Figur 2 durchgeführt werden, welches ein anfängliches Kalibrieren S1 sowie ein erneutes Kalibrieren S2 umfasst, wobei beispielsweise das anfängliche Kalibrieren S1 werkseitig vor einem Ausliefern der entsprechenden Analyseeinheit 10 stattfinden kann, während das erneute Kalibrieren S2 im Feld bei bereits installierten und im Betrieb befindlichen Analyseeinheiten durchgeführt werden kann. Das entsprechende Verfahren kann für jede der Sensoreinheiten 12a bis 12c gesondert durchgeführt werden, oder es können mehrere der Sensoreinheiten 12a bis 12c gleichzeitig kalibriert werden.

Hierbei umfasst das anfängliche Kalibrieren S1 zunächst ein Ermitteln einer anfänglichen Nullpunktausgabe der entsprechenden Sensoreinheit mit einem ersten Kalibrier-Gasgemisch, in welchem die Konzentration des vorbestimmten Gases im Wesentlichen Null beträgt, bei einem Schritt S11. Hierbei kann daran gedacht werden, die Ermittlung der anfänglichen Nullpunkt-Ausgabe sämtlicher der Sensoreinheiten 12a bis 12c gleichzeitig durchzuführen, sofern ein entsprechendes Kalibrier-Gasgemisch zur Verfügung steht, in welchem die Konzentration sämtlicher der durch die Sensoreinheiten 12a bis 12c zu ermittelnden Gase jeweils Null beträgt.

Anschließend wird in Schritt S12 eine weitere Ausgabe der entsprechenden wenigstens einen Sensoreinheit mit einem zweiten Kalibrier-Gasgemisch ermittelt, in welchem die Konzentration des vorbestimmten Gases kleiner oder vorzugsweise größer als die Grenzwert-Konzentration ist. Auch hierbei kann eine parallele Ermittlung von weiteren Ausgaben einer Mehrzahl der Sensoreinheiten 12a bis 12c stattfinden, sofern entsprechende Kalibrier-Gasgemische verfügbar sind, welche mehrere der einzelnen Gase in geeigneten Konzentrationen enthalten, und wenn die verwendeten Sensoreinheiten 12a bis 12c keine Querempfindlichkeiten zeigen. Hierbei sei darauf verwiesen, dass der Schritt S12 auch mehrfach mit unterschiedlichen Gasgemischen durchgeführt werden kann, in welchen die Konzentration des vorbestimmten Gases jeweils unterschiedlich, aber größer Null ist, worauf hin dann im Folgenden eine Mehrzahl entsprechender Werte zu verarbeiten sind.

Anschließend wird in Schritt S13 eine Ausgabe-Gerade der entsprechenden Sensoreinheit anhand der in den Schritten S11 und S12 ermittelten Nullpunkt-Ausgabe und der wenigstens einen weiteren Ausgabe durchgeführt, wobei die Ausgabe-Gerade eine anfängliche Geradensteigung α aufweist. Diese anfängliche Ausgabe-Gerade wird nach einer ersten Inbetriebnahme der entsprechenden Sensoreinheit verwendet, wobei zu erwarten ist, dass sich die Eigenschaften der Sensoreinheiten 12a bis 12c mit der Zeit hinsichtlich ihrer Sensorausgaben ändern werden und demzufolge eine erneute Kalibrierung in regelmäßigen Zeitabständen notwendig sein wird.

Zu diesem Zweck kann bei dem erneuten Kalibrieren S2 eine Grenzwert-Ausgabe der entsprechenden Sensoreinheit mit einem Grenzwert-Gasgemisch bei Schritt S21 ermittelt werden, in welchem die Konzentration des vorbestimmten Gases im Wesentlichen einer Grenzwert-Konzentration in der oben beispielhaft anhand von Atemluft beschriebenen Weise entspricht. Auch an dieser Stelle kann das Grenzwert-Gasgemisch erneut mehrere der zu erfassenden Gase in ihren jeweiligen Grenzwert-Konzentrationen enthalten, sodass erneut eine Ermittlung von Grenzwert-Ausgaben von einer Mehrzahl der Sensoreinheiten 12a bis 12c gleichzeitig durchgeführt werden kann.

Anschließend wird beim Schritt S22 eine aktualisierte Ausgabe-Gerade der entsprechenden Sensoreinheit anhand der neu ermittelten Grenzwert-Ausgabe und der anfänglichen Nullpunkt-Ausgabe oder anhand der Grenzwert-Ausgabe und der anfänglichen Geradensteigung ermittelt. Hierdurch kann sichergestellt werden, dass in der Umgebung der entsprechenden Grenzwert-Konzentration des mittels der jeweiligen Sensoreinheit zu erfassenden Gases in einem abzufüllenden Gasgemisch eine hohe Genauigkeit hinsichtlich dessen erzielt wird, ob der Grenzwert überschritten oder unterschritten ist.

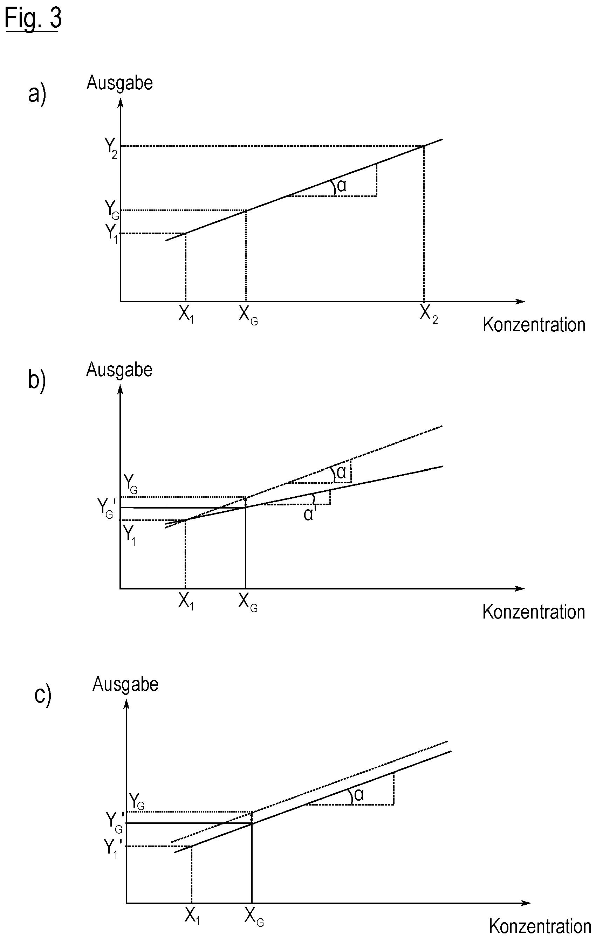

Das in der Anordnung aus Figur 1 in der in Figur 2 dargestellten Weise durchgeführte Verfahren sei nun zuletzt noch anhand der Schaubilder in Figur 3 beispielhaft erläutert. Hierbei zeigt das Schaubild a) eine Sensor-Ausgabe auf der y-Achse, welche über einer Konzentration eines entsprechenden Gases in einem zu untersuchenden Gasgemisch auf der x-Achse aufgetragen ist. Der Sensorrohwert kann hierbei je nach verwendetem Sensortyp beispielsweise eine Spannung in Millivolt (mV) oder ein Strom in Milliampere (mA) sein. Die beiden auf der x-Achse markierten Werte X₁ und X₂ entsprechen hierbei einer Konzentration von 0 bzw. einer Konzentration, welche eine obere Grenze des Messbereichs der entsprechenden Sensoreinheit darstellt. Ferner ist eine Grenzwert-Konzentration durch den x-Wert X_{G} markiert. An den Stellen X₁ und X₂ kann nunmehr bei der anfänglichen Kalibrierung der entsprechenden Sensoreinheit jeweils eine Nullpunkt-Ausgabe und eine weitere Ausgabe der Sensoreinheit ermittelt werden, welche dementsprechend den Ausgebewerten Y₁ und Y₂ entsprechen. Indem durch die Punkte (X₁, Y₁) und (X₂, Y₂) eine Gerade mit der bereits angesprochenen Steigung α gelegt wird, kann die der Grenzwert-Konzentration X_{G} zugeordnete Grenzwertausgabe Y_{G} abgeleitet werden.

Es ist nun bei üblicherweise verwendeten Sensoreinheiten zu erwarten, dass sich im Laufe der Zeit die auf der y-Achse dargestellten Ausgaben für gegebene Konzentrationen auf der x-Achse ändern und insbesondere zurückgehen. Demzufolge ist in der oben beschriebenen Weise eine erneute Kalibrierung nach einer gewissen Zeitspanne oder gemäß einer bestimmten Zeitperiode notwendig.

Da die Sensoreinheiten 12a bis 12c in dem hier diskutierten Anwendungsfall in erster Linie eine Unterschreitung oder Überschreitung der Grenzwert-Konzentration X_{G} zu überwachen haben, kann nun erfindungsgemäß mittels einer Grenzwert-Einpunkt-Kalibrierung bei dem Konzentrationswert X_{G} eine solche Nachkalibrierung in einfacher und effizienter Weise stattfinden, indem die Grenzwert-Ausgabe Y_{G}' in der beschriebenen Weise bestimmt wird. Die beiden möglichen Wege zum Ableiten einer entsprechenden aktualisierten Ausgabe-Gerade lassen sich anhand der Schaubilder b) und c) in Figur 3 nachvollziehen.

Hierbei ist jeweils die Sensorausgabe Y_{G}' bei der Grenzwert-Konzentration X_{G} geringer als die in Figur 1 extrapolierte Grenzwertausgabe Y_{G}. Um nun dennoch eine hohe Genauigkeit im Bereich der Grenzwert-Konzentration X_{G} nach der erneuten Kalibrierung sicherstellen zu können, ist in Schaubild b) anhand der bei dem erneuten Kalibrieren ermittelten Grenzwert-Ausgabe Y_{G}' bei der Grenzwert-Konzentration X_{G} und der anfänglichen Nullpunkt-Ausgabe Y₁ an dem Nullpunkt X₁ eine aktualisierte Ausgabe-Gerade mit einer Steigung α' gebildet worden, welche eine geringere Steigung als die in Schaubild b) gestrichelt dargestellte ursprüngliche Ausgabe-Gerade aufweist. Dennoch kann in der Umgebung der Grenzwert-Konzentration X_{G} mit einer hohen Genauigkeit ein Überschreiten oder Unterschreiten der Grenzwert-Konzentration X_{G} auf Grundlage einer entsprechenden Sensor-Ausgabe festgestellt werden.

In ähnlicher Weise wurde in dem Schaubild c) auf Grundlage der bei dem erneuten Kalibrieren festgestellten Grenzwert-Ausgabe Y_{G}' bei der Grenzwert-Konzentration X_{G} sowie der Steigung α der bei dem anfänglichen Kalibrieren ermittelten Ausgabe-Gerade eine aktualisierte Ausgabe-Gerade gebildet, welche dementsprechend parallel zu der in Schaubild c) gestrichelt eingezeichneten ursprünglichen Ausgabe-Gerade verläuft. Auch durch das Bilden dieser aktualisierten Ausgabe-Gerade kann sichergestellt werden, dass in der Umgebung der Grenzwert-Konzentration X_{G} mit hoher Präzision ein Überschreiten oder Unterschreiten davon festgestellt werden kann, wenngleich beispielsweise in diesem Fall die extrapolierte Nullpunkt-Ausgabe Y₁' von der ursprünglich ermittelten Nullpunkt-Ausgabe Y₁ abweicht.

Dementsprechend kann durch die Grenzwert-Einpunkt-Kalibrierung bei dem erneuten Kalibrieren gemäß den Schaubildern b) und c) mit vermindertem Aufwand dennoch der Verringerung der Sensorausgabe über der Zeit Rechnung getragen werden, während gleichzeitig mit hoher Genauigkeit festgestellt werden kann, ob eine Konzentration eines zu erfassenden Gases in einem Gasgemisch oberhalb oder unterhalb eines vorbestimmten Grenzwerts liegt.

## Patentansprüche

1. Verfahren zum Kalibrieren wenigstens einer Sensoreinheit (12a - 12c), welche für ein Bestimmen einer Konzentration eines vorbestimmten Gases in einem Gasgemisch vorgesehen ist, wobei eine Grenzwert-Konzentration (X_{G}) vorgegeben ist, welche durch das vorbestimmte Gas in dem Gasgemisch nicht überschritten werden darf, umfassend:
anfängliches Kalibrieren (S1) der Sensoreinheit (12a - 12c) bei wenigstens zwei vorbestimmten Konzentrationen (X₁, X₂) des vorbestimmten Gases in einem jeweiligen Kalibier-Gasgemisch, wenigstens umfassend die Schritte:
- Ermitteln (S11) einer anfänglichen Nullpunkt-Ausgabe (Y₁) der Sensoreinheit (12a - 12c) mit einem ersten Kalibrier-Gasgemisch, in welchem die Konzentration (X₁) des vorbestimmten Gases im Wesentlichen Null beträgt,
- Ermitteln (S12) einer weiteren Ausgabe (Y₂) der Sensoreinheit (12a - 12c) mit einem zweiten Kalibrier-Gasgemisch, in welchem die Konzentration (X₂) des vorbestimmten Gases größer als die Grenzwert-Konzentration (X_{G}) ist, und
- Bilden (S13) einer Ausgabe-Gerade der Sensoreinheit (12a - 12c) anhand der anfänglichen Nullpunkt-Ausgabe (Y₁) und der weiteren Ausgabe (Y₂), wobei die Ausgabe-Gerade eine anfängliche Geradensteigung (α) aufweist;
**dadurch gekennzeichnet, dass** das Verfahren zu einem späteren Zeitpunkt ein erneutes Kalibrieren (S2) umfasst, umfassend die Schritte:
- Ermitteln (S21) einer Grenzwert-Ausgabe (Y_{G}') der Sensoreinheit (12a - 12c) mit einem Grenzwert-Gasgemisch, in welchem die Konzentration (X_{G}) des vorbestimmten Gases im Wesentlichen der Grenzwert-Konzentration (X_{G}) entspricht, und
- Bilden (S22) einer aktualisierten Ausgabe-Gerade der Sensoreinheit (12a - 12c) anhand der Grenzwert-Ausgabe (Y_{G}') und der anfänglichen Nullpunkt-Ausgabe (Y₁) oder anhand der Grenzwert-Ausgabe (Y_{G}') und der anfänglichen Geradensteigung (α).

2. Verfahren nach Anspruch 1,
nach dem Bilden der aktualisierten Ausgabe-Gerade ferner umfassend einen Schritt eines Überprüfens, ob die Geradensteigung (α') der aktualisierten Ausgabe-Gerade und/oder ein Nullpunkt-Wert (Y₁') der aktualisierten Ausgabe-Gerade in einem vorbestimmten Wertebereich liegt.

3. Verfahren nach Anspruch 1 oder 2,
wobei in dem zweiten Kalibrier-Gasgemisch das vorbestimmte Gas mit einer Konzentration (X₂) vorliegt, welche einer oberen Grenze des Messbereichs der entsprechenden Sensoreinheit (12a - 12c) entspricht.

4. Verfahren nach einem der vorhergehenden Ansprüche,
wobei das anfängliche Kalibrieren umfasst:
- Ermitteln mehrerer weiterer Ausgaben (Y₂) mit unterschiedlichen Gasgemischen, in welchen die Konzentration (X₂) des vorbestimmten Gases jeweils unterschiedlich und größer Null ist,
- Bilden einer Ausgleich-Ausgabe-Gerade der Sensoreinheit (12a - 12c) anhand der anfänglichen Nullpunkt-Ausgabe (Y₁) und der mehreren weiteren Ausgaben (Y₂).

5. Verfahren nach einem der vorhergehenden Ansprüche,
wobei während des anfänglichen und/oder erneuten Kalibrierens gleichzeitig eine Mehrzahl von Sensoreinheiten (12a - 12c) kalibriert werden, indem die jeweiligen Gasgemische eine entsprechende Mehrzahl von zu bestimmenden Gasen in geeigneten Konzentrationen enthalten.

6. Verfahren nach einem der vorhergehenden Ansprüche,
wobei das Gasgemisch Atemluft ist und/oder das vorbestimmte Gas CO, CO₂ oder O₂ ist.

7. Verfahren nach einem der vorhergehenden Ansprüche,
wobei nach dem anfänglichen Kalibrieren und/oder dem erneuten Kalibrieren ein Prüfen der Kalibrierung mit einem Prüf-Gasgemisch durchgeführt wird, in welchem die Konzentration des vorbestimmten Gases geringfügig über der Grenzwert-Konzentration liegt.

8. Verfahren nach einem der vorhergehenden Ansprüche,
wobei während des anfänglichen oder erneuten Kalibrierens und/oder des Prüfens ein von der wenigstens einen Sensoreinheit (12a - 12c) überwachter Kompressor (20) abgeschaltet wird, ein Spülventil geöffnet wird und/oder eine Anzeige für einen Bediener angepasst wird.

9. Verfahren nach einem der vorhergehenden Ansprüche,
ferner umfassend ein Überwachen einer Durchflussrate des jeweiligen Gasgemisches durch die wenigstens eine Sensoreinheit (12a - 12c).

10. Verfahren nach dem vorhergehenden Anspruch,
wobei bei einem Unterschreiten und/oder Überschreiten einer Grenzwert-Durchflussrate für einen vorbestimmten Zeitraum eine Warnung ausgegeben wird oder ein automatischer Abbruch der Kalibrierung erfolgt.

11. Analyseeinheit (10) für Gasgemische, umfassend:
- wenigstens eine Sensoreinheit (12a - 12c), welche dazu eingerichtet ist, Sensordaten auszugeben, welche eine Konzentration eines vorbestimmten Gases in dem Gasgemisch repräsentieren; und
- eine Steuereinheit (14), welche mit der wenigstens einen Sensoreinheit (12a - 12c) betriebsmäßig gekoppelt und in einem Arbeitsmodus dazu eingerichtet ist, anhand der Sensordaten die Konzentration des vorbestimmten Gases zu ermitteln;
wobei die Steuereinheit (14) dazu eingerichtet ist, zwischen dem Arbeitsmodus und einem Kalibriermodus umschaltbar zu sein und in dem Kalibriermodus die Schritte eines Verfahrens nach einem der vorhergehenden Ansprüche durchzuführen.

12. Analyseeinheit (10) nach Anspruch 11,
wobei die Steuereinheit (14) ferner dazu eingerichtet ist, in dem Arbeitsmodus bei einem Feststellen eines Überschreitens einer Grenzwert-Konzentration des vorbestimmten Gases der wenigstens einen Sensoreinheit (12a - 12c) einen Steuerbefehl zum Öffnen eines Spülventils oder eines Abschalten eines Kompressors (20) auszugeben.

13. Analyseeinheit (10) nach einem der Ansprüche 11 und 12,
wobei die Analyseeinheit (10) mit einem Gasspeicher bereitgestellt ist, welcher ein Prüf-Gasgemisch enthält, in welchem das wenigstens vorbestimmte Gas geringfügig oberhalb der Grenzwert-Konzentration (X_{G}) vorliegt, und die Steuereinheit (14) dazu eingerichtet ist, nach einer vorbestimmten Zeitspanne oder bei einer erneuten Inbetriebnahme der Analyseeinheit (10) automatisch in einen Prüfmodus umzuschalten und eine Prüfung der wenigstens einen Sensoreinheit (12a - 12c) mithilfe des in dem Gasspeicher enthaltenen Prüf-Gasgemischs durchzuführen.

14. Analyseeinheit (10) nach einem der Ansprüche 11 bis 13,
wobei die oder wenigstens eine der Sensoreinheiten (12a - 12c) eine elektrochemische Sensoreinheit ist.

15. Kompressor (20) zum Verdichten von Atemluft für eine Abfüllung in einen Druckgasspeicher (S), umfassend eine Analyseeinheit (10) nach einem der Ansprüche 11 bis 14, welche dazu eingerichtet ist, während eines Betriebs des Kompressors (20) die ausgegebene komprimierte Atemluft auf eine Konzentration von wenigstens einem vorbestimmten Gas zu überwachen, wobei vorzugsweise die Steuereinheit (14) der Analyseeinheit (10) mit einer Steuereinheit (22) des Kompressors (20) betriebsmäßig gekoppelt oder integral mit ihr gebildet ist.

## Claims

1. Method for calibrating at least one sensor unit (12a - 12c) which is provided for determining a concentration of a predetermined gas in a gas mixture, wherein a limit value concentration (X_{G}) is specified which must not be exceeded by the predetermined gas in the gas mixture, comprising:
initial calibration (S1) of the sensor unit (12a - 12c) at at least two predetermined concentrations (X₁, X₂) of the predetermined gas in a respective calibration gas mixture, at least comprising the steps:
- Determining (S11) an initial zero point output (Y₁) of the sensor unit (12a - 12c) with a first calibration gas mixture in which the concentration (X₁) of the predetermined gas is substantially zero,
- Determining (S12) a further output (Y₂) of the sensor unit (12a - 12c) with a second calibration gas mixture in which the concentration (X₂) of the predetermined gas is greater than the limit value concentration (X_{G}), and
- Forming (S13) an output straight line of the sensor unit (12a - 12c) based on the initial zero point output (Y₁) and the further output (Y₂), wherein the output straight line has an initial line slope (α);
**characterized in that** the method comprises a recalibration (S2) at a later point in time, comprising the steps:
- Determining (S21) a limit value output (Y_{G}') of the sensor unit (12a - 12c) with a limit value gas mixture in which the concentration (X_{G}) of the predetermined gas substantially corresponds to the limit value concentration (X_{G}), and
- Forming (S22) an updated output straight line of the sensor unit (12a - 12c) based on the limit value output (Y_{G}') and the initial zero point output (Y₁) or based on the limit value output (Y_{G}') and the initial straight line slope (α).

2. Method according to claim 1, furthermore comprising, after forming the updated output line, a step of checking whether the line slope (α') of the updated output line and/or a zero point value (Y₁') of the updated output line lies in a predetermined value range.

3. Method according to claim 1 or 2,
wherein in the second calibration gas mixture the predetermined gas is present at a concentration (X₂) which corresponds to an upper limit of the measuring range of the corresponding sensor unit (12a - 12c).

4. Method according to one of the preceding claims,
where the initial calibration includes:
- Determining several further outputs (Y₂) with different gas mixtures, in which the concentration (X₂) of the predetermined gas is in each case different and greater than zero,
- Forming a compensation output line of the sensor unit (12a - 12c) based on the initial zero point output (Y₁) and the several further outputs (Y₂).

5. Method according to one of the preceding claims,
wherein during the initial and/or recalibration a plurality of sensor units (12a - 12c) are calibrated simultaneously, in that the respective gas mixtures contain a corresponding plurality of gases to be determined in suitable concentrations.

6. Method according to one of the preceding claims,
wherein the gas mixture is breathing air and/or the predetermined gas is CO, CO₂ or O₂.

7. Method according to one of the preceding claims,
wherein after the initial calibration and/or the recalibration, a calibration check is carried out with a test gas mixture in which the concentration of the predetermined gas is slightly above the limit value concentration.

8. Method according to one of the preceding claims, wherein during the initial calibration or recalibration and/or testing, a compressor (20) monitored by the at least one sensor unit (12a - 12c) is switched off, a flush valve is opened and/or a display for an operator is adjusted.

9. Method according to one of the preceding claims,
further comprising a monitoring of a flow rate of the respective gas mixture by the at least one sensor unit (12a - 12c).

10. Method according to the preceding claim,
wherein if the flow rate falls below and/or exceeds a limit value for a predetermined period of time, a warning is issued or the calibration is automatically stopped.

11. Analysis unit (10) for gas mixtures, comprising:
- at least one sensor unit (12a - 12c), which is designed to output sensor data representing a concentration of a predetermined gas in the gas mixture; and
- a control unit (14), which is operatively coupled to the at least one sensor unit (12a - 12c) and is designed to determine in an operating mode the concentration of the predetermined gas based on the sensor data;
wherein the control unit (14) is designed to be switchable between the working mode and a calibration mode and to carry out the steps of a method according to one of the preceding claims in the calibration mode.

12. Analysis unit (10) according to claim 11,
wherein the control unit (14) is further designed to issue in the operating mode a control command to open a flush valve or switch off a compressor (20) if it detects that a limit value concentration of the predetermined gas of the at least one sensor unit (12a - 12c) has been exceeded.

13. Analysis unit (10) according to one of claims 11 and 12,
wherein the analysis unit (10) is provided with a gas reservoir containing a test gas mixture in which the at least predetermined gas is present in a concentration slightly above the limit value concentration (X_{G}), and
the control unit (14) is designed to switch automatically to a test mode after a predetermined period of time or when the analysis unit (10) is restarted, and to carry out a test of the at least one sensor unit (12a - 12c) with the aid of the test gas mixture contained in the gas reservoir.

14. Analysis unit (10) according to one of claims 11 to 13,
wherein the or at least one of the sensor units (12a - 12c) is an electrochemical sensor unit.

15. Compressor (20) for compressing breathing air for filling into a compressed gas reservoir (S), comprising an analysis unit (10) according to one of claims 11 to 14, which is designed to monitor the output compressed breathing air for a concentration of at least one predetermined gas during operation of the compressor (20), wherein preferably the control unit (14) of the analysis unit (10) is operatively coupled to or is formed integrally with a control unit (22) of the compressor (20).

## Revendications

1. Procédé d'étalonnage d'au moins une unité de capteur (12a - 12c), qui est destinée à déterminer une concentration d'un gaz prédéterminé dans un mélange gazeux, dans lequel une concentration à valeur limite (X_{G}) est spécifiée, qui ne doit pas être dépassée par le gaz prédéterminé dans le mélange gazeux, comprenant : un étalonnage initial (S1) de l'unité de capteur (12a - 12c) à au moins deux concentrations (X₁, X₂) prédéterminées du gaz prédéterminé dans un mélange gazeux d'étalonnage respectif, comprenant au moins les étapes :
• d'établissement (S11) d'une sortie de point zéro initiale (Y₁) de l'unité de capteur (12a - 12c) avec un premier mélange gazeux d'étalonnage dans lequel la concentration (X₁) du gaz prédéterminé est essentiellement nulle,
• d'établissement (S12) d'une sortie supplémentaire (Y₂) de l'unité de capteur (12a - 12c) avec un second mélange gazeux d'étalonnage dans lequel la concentration (X₂) du gaz prédéterminé est supérieure à la concentration à valeur limite (X_{G}), et
• de formation (S13) d'une droite de sortie de l'unité de capteur (12a - 12c) sur la base de la sortie de point zéro initiale (Y₁) et de la sortie supplémentaire (Y₂), dans lequel la droite de sortie présente une pente de droite initiale (a) ;
**caractérisé en ce que** le procédé comprend, à un instant ultérieur, un réétalonnage (S2), comprenant les étapes :
• d'établissement (S21) d'une sortie à valeur limite (Y_{G}') de l'unité de capteur (12a - 12c) avec un mélange gazeux à valeur limite dans lequel la concentration (X_{G}) du gaz prédéterminé correspond essentiellement à la concentration à valeur limite (X_{G}), et
• de formation (S22) d'une droite de sortie mise à jour de l'unité de capteur (12a - 12c) sur la base de la sortie à valeur limite (Y_{G}') et de la sortie de point zéro initiale (Y₁) ou sur la base de la sortie à valeur limite (Y_{G}') et de la pente de droite initiale (a).

2. Procédé selon la revendication 1, comprenant en outre, après la formation de la droite de sortie mise à jour, une étape consistant à vérifier si la pente de droite (a') de la droite de sortie mise à jour et/ou une valeur de point zéro (Y₁') de la droite de sortie mise à jour se situe dans une plage de valeurs prédéterminée.

3. Procédé selon la revendication 1 ou 2, dans lequel, dans le second mélange gazeux d'étalonnage, le gaz prédéterminé est présent avec une concentration (X₂) qui correspond à une limite supérieure de la plage de mesure de l'unité de capteur (12a - 12c) correspondante.

4. Procédé selon l'une quelconque des revendications précédentes, dans lequel l'étalonnage initial comprend :
• l'établissement de plusieurs sorties supplémentaires (Y₂) avec différents mélanges gazeux dans lesquels la concentration (X₂) du gaz prédéterminé est à chaque fois différente et supérieure à zéro,
• la formation d'une droite de sortie de compensation de l'unité de capteur (12a - 12c) sur la base de la sortie de point zéro initiale (Y₁) et des plusieurs sorties supplémentaires (Y₂).

5. Procédé selon l'une quelconque des revendications précédentes, dans lequel, pendant l'étalonnage initial et/ou le réétalonnage, une pluralité d'unités de capteur (12a - 12c) sont étalonnées simultanément, du fait que les mélanges gazeux respectifs contiennent une pluralité correspondante de gaz à déterminer dans des concentrations appropriées.

6. Procédé selon l'une quelconque des revendications précédentes, dans lequel le mélange gazeux est de l'air respirable et/ou le gaz prédéterminé est du CO, du CO₂ ou de l'O₂.

7. Procédé selon l'une quelconque des revendications précédentes, dans lequel, après l'étalonnage initial et/ou le réétalonnage, un test de l'étalonnage est effectué avec un mélange gazeux de test, dans lequel la concentration du gaz prédéterminé est légèrement supérieure à la concentration à valeur limite.

8. Procédé selon l'une quelconque des revendications précédentes, dans lequel, pendant l'étalonnage initial ou le réétalonnage et/ou le test, un compresseur (20) surveillé par l'au moins une unité de capteur (12a - 12c) est arrêté, une vanne de purge est ouverte et/ou un affichage pour un opérateur est adapté.

9. Procédé selon l'une quelconque des revendications précédentes, comprenant en outre la surveillance d'un débit du mélange gazeux respectif à travers l'au moins une unité de capteur (12a - 12c).

10. Procédé selon la revendication précédente, dans lequel, en cas de passage en dessous et/ou de dépassement d'un débit à valeur limite pendant une période prédéterminée, un avertissement est émis ou un arrêt automatique de l'étalonnage a lieu.

11. Unité d'analyse (10) pour mélanges gazeux, comprenant :
• au moins une unité de capteur (12a - 12c), qui est configurée pour émettre des données de capteur qui représentent une concentration d'un gaz prédéterminé dans le mélange gazeux ; et
• une unité de commande (14), qui est couplée de manière opérationnelle à l'au moins une unité de capteur (12a-12c) et est configurée dans un mode de fonctionnement pour établir la concentration du gaz prédéterminé sur la base des données de capteur ;
dans laquelle l'unité de commande (14) est configurée pour pouvoir basculer entre le mode de fonctionnement et un mode d'étalonnage et pour mettre en œuvre dans le mode d'étalonnage les étapes d'un procédé selon l'une quelconque des revendications précédentes.

12. Unité d'analyse (10) selon la revendication 11, dans laquelle l'unité de commande (14) est en outre configurée pour, dans le mode de fonctionnement, lors de la détection d'un dépassement d'une concentration à valeur limite du gaz prédéterminé de l'au moins une unité de capteur (12a - 12c), émettre une instruction de commande pour l'ouverture d'une vanne de purge ou pour l'arrêt d'un compresseur (20).

13. Unité d'analyse (10) selon l'une quelconque des revendications 11 et 12, dans laquelle l'unité d'analyse (10) est pourvue d'un réservoir de gaz, qui contient un mélange gazeux de test dans lequel l'au moins un gaz prédéterminé est présent légèrement au-dessus de sa concentration à valeur limite (X_{G}), et l'unité de commande (14) est configurée pour, après un laps de temps prédéterminé ou lors d'une nouvelle mise en service de l'unité d'analyse (10), basculer automatiquement dans un mode de test et mettre en œuvre un test de l'au moins une unité de capteur (12a - 12c) au moyen du mélange gazeux de test contenu dans le réservoir de gaz.

14. Unité d'analyse (10) selon l'une quelconque des revendications 11 à 13, dans laquelle l'une ou au moins l'une des unités de capteur (12a - 12c) est une unité de capteur électrochimique.

15. Compresseur (20) pour la compression d'air respirable pour le remplissage dans un réservoir de gaz sous pression (S), comprenant une unité d'analyse (10) selon l'une quelconque des revendications 11 à 14, qui est configurée pour surveiller, pendant un fonctionnement du compresseur (20), l'air respirable comprimé sorti à une concentration d'au moins un gaz prédéterminé, dans lequel de préférence l'unité de commande (14) de l'unité d'analyse (10) est couplée de manière opérationnelle à une unité de commande (22) du compresseur (20) ou est formée d'un seul tenant avec celle-ci.
